# EUROPEAN PATENT SPECIFICATION

(11) **EP 1 942 951 B1**
(45) Date of publication and mention of the grant of the patent: **12.12.2012**
(21) Application number: 06807542.3
(22) Date of filing: 25.10.2006
(51) Int. Cl.: A61L 2/18, C02F 1/46

(54) **METHOD FOR THE PREPARATION OF BIOCIDAL ACTIVATED WATER SOLUTIONS**
METHODE ZUR HERSTELLUNG VON BIOZIDEN AKTIVIERTEN WASSERLÖSUNGEN
MÉTHODE POUR LA PRÉPARATION DES SOLUTIONS ACTIVÉES ET BIOCIDES D'EAU

(30) Priority: 25.10.2005 EP 05109974
(43) Date of publication of application: 16.07.2008
(73) Proprietor: Aseptix Technologies B.V, 3632 AX Loenen aan de Vecht (NL)
(72) Inventor: BOBBERT, Ilja, NL-1213 RT Hilversum (NL)
(74) Representative: van Heuvel, Margaretha
(86) International application number: PCT/EP2006/067765
(87) International publication number: WO 2007/048806

(56) References cited:
- WO-A-02/059046
- WO-A-2005/097213
- DE-A1- 19 951 117
- US-A- 5 478 533
- US-A1- 2004 069 618

## Description

The present invention relates to a method for the production of activated water.

US Patent 5,464,513 discloses a method and apparatus for water decontamination using a series of pulsed electric arc discharges. The method comprises the positioning of both electrodes in a water stream; and treating said water with a series of pulsed electric arc discharges to generate mechanical shock waves and pulses of lethal ultraviolet radiation.

US Patent 5,876,663 discloses a method for sterilization of a liquid which contains microorganisms using plasma glow discharge. The method consists of placing the liquid to be processed in a container at a depth of between 0.1 and 2 cm at atmospheric pressure. Two electrodes are then positioned in such a way that the first electrode is located under the bottom of the container and the second electrode is located above the surface of the liquid in the container. The liquid to be processed is then exposed to a steady-state glow discharge plasma at an RF voltage of between 1 and 5 kV and a frequency in the range of 1-100 kHz.

WO 2005/097213 relates to a device and a method for purifying liquids with the aid of radicals that are produced by means of electron transfer. Said electrons can be formed by plasmas, an electron beam, corona discharge, and electromagnetic waves, which are generated directly at the boundary layer between the liquid that is to be purified and a gas. The method is suitably especially for decomposing substances in liquids, such as endocrinally active substances, bioactive substances, environmental pollutants, pesticides, herbicides, X-ray contrast media, medicaments, solvents, and other substances.

Such methods do not provide teaching with regard to conditions that are useful for the production of activated water having biocidal activity:

Methods and devices for treatment or activation of chemically pure and potable water by applying a non-self maintained glow discharge plasma between two unlike electrodes, have been disclosed in WO 02/059045 and WO 02/059046. The method comprises having a turbulent flow of treated water through a reaction chamber consisting of several annular shaped electrodes, placed coaxially, applying very high current strengths (0.1-20 A), using a low voltage (not above 500 V), and applying a rather high clock frequency (0.1-100 kHz). In addition, a magnetic field in the discharge zone is established to increase efficiency. Finally, reference is made to a duty cycle of at least 1.3 and examples are shown with duty cycles (also referred to as "relative pulse duration") of 2, 3 and 4. However the Applicant was not able to interpret a duty cycle larger than 1, as the definition of a duty cycle is the ratio of the effective pulse train to the pulse duration, which by definition is a relative number always equal to or lower than 1. The main disadvantages of this method further are the high operating current (see examples), the high clock frequency causing very high power consumption and the fact that the form of the electrodes combined with the very thin water sheet easily forms arcs and does not provide for suitable process control.

It is objective of this invention to provide an improved method for obtaining a biocidal solution which is nontoxic and has strong biocidal properties.

Thus, in a first aspect, the present invention provides a method of producing a biocidal activated aqueous solution. In the method of the invention, a particular glow discharge plasma is used.

A preferred method of producing the activated aqueous solution of the present invention comprises:
(a) placing an aqueous solution in the form of a layer in a chamber suitable for a glow discharge plasma treatment,
(b) positioning an electrode in the layer of (a) and an electrode with opposite potential in the gas phase above said layer,
(c) generating a glow discharge plasma in the chamber, and
(d) subjecting the aqueous solution to said glow discharge plasma at a voltage of 1000 10,000 V and a current per electrode of 20-500 mA, to obtain an activated aqueous solution.

The aqueous solution used in step (a), sometimes called herein "initial aqueous solution", may be tap water, potable water, demineralized, distilled and/or chemically pure water, preferably is demineralized, distilled and/or chemically pure water.

In one embodiment of the invention, the initial aqueous solution may contain an additive. For instance, an additive that is able to increase activation efficiency and/or biocidal activity of the resulting activated aqueous solution. Among the additives that may be added to the water prior to its treatment is a lower alcohol of the formula ROH, wherein R is a straight or branched C₁-C₆ alkyl, or a mixture thereof. In a preferred embodiment, the additive is methanol or ethanol, or a mixture thereof.

The lower alcohol is preferably added at a concentration of at the most 1% (w/w), more preferably 0.05 to 0.5% (w/w), most preferably 0.1% (w/w).

The initial aqueous solution may be at any pH, there is no preferred pH range prior to the treatment of the solution according to the invention. The pH of the initial aqueous solution may vary depending on the type of water that is used.

The initial aqueous solution is subjected to the glow discharge plasma or the corona discharge as described in this invention to obtain an activated aqueous solution.

The initial aqueous solution is placed in the form of a layer in a suitable chamber. Preferably, the layer has a depth from about 10 to 200 mm. The aqueous layer may be static or flowing, and oriented vertically, horizontally or inclined. In case of a vertical or inclined layer, the process is operated using a flow reactor, in which the water layer is flowing over one electrode and undergoing a discharge treatment from a discharge extending to the opposite electrode. Preferably, the layer is static and/or oriented horizontally, allowing the most convenient process. It is possible to bubble air through the aqueous layer to improve the activation process.

The depth of the aqueous layer should take into account that the distance between the top electrode and the liquid surface should not exceed a distance of about 15 mm.

The chamber may be any chamber, e.g. a cell or vessel, which is suitable for plasma production and treatment of an aqueous solution according to the invention.

Unlike electrodes (anode and cathode) are placed in the chamber. As used herein the term "unlike electrodes" refers to a set of electrodes of opposite potential, one of which is positive (anode) and one of which is negative (cathode). An electrode is positioned in the aqueous layer and an opposite electrode in the gas phase above the aqueous layer. Various embodiments for the set of unlike electrodes exist. For instance, the unlike electrodes may consist of one pair of anode and cathode or of multiple pairs of anode and cathode. It is also possible that one electrode of the unlike electrodes consists of multiple electrodes, discharging to one opposite electrode. Preferably, multiple anodes may discharge to one cathode.

In one embodiment, the positive electrode or anode is positioned in the gas phase above the surface of the liquid and the negative electrode or cathode is positioned below the surface of the liquid. It has been found that this provides stable operation and requires less current than vice versa. In this case, the surface of the liquid also acts as an electrode.

It is also possible that the lower electrode is positioned outside and underneath the reaction chamber.

The electrodes that can be used in accordance with the method of this invention can be comprised of any material that does not have a catalytic effect on hydrogen peroxide and super-peroxides and has high electrical conductivity. Examples of electrode materials include, but are not limited to, metals such as tungsten, titanium, tantalum, vanadium, zirconium, tin, stainless steel. Any other electrodes, which would be known to those in the art to satisfy the conditions necessary for water activation using a non-thermal electrical discharge, are also contemplated for use in the present invention.

In a preferred embodiment, the electrodes, more preferably the anode(s), are (is) of a pin shape and comprise(s) a relatively sharp tip, to ensure a high potential gradient, to enhance activation efficiency, to prevent arcing and to maintain a stable discharge. With a relatively sharp tip is meant that the point of the tip has at the most a diameter which is 1/3 of the diameter of the electrode, preferably a smaller diameter. More preferably the tip is a needle-sharp tip. The top angle of the pin-shaped electrode preferably is about 45°.

In a system, where two phases (fluid (gas) and liquid) are present, and an electric current is being formed between an electrode immersed in the liquid phase and an electrode placed into the gas phase, the boundary of the two phases is of special importance because this is where intensive physical and chemical transformations are taking place, which in turn drive the reactions taking place in the water. Thus, the thickness of the water layer and the distance between electrodes are critical for providing uniform treatment the aqueous layer, and thus to increase efficiency of activation of the aqueous layer. The distance between the electrode in the gas phase (top electrode) and the liquid surface preferably is from about 3 - 15 mm, more preferably from about 3 - 12 mm, most preferably about 8-10 mm.

The pressure in the chamber should enable the formation of a uniform glow discharge plasma and may be atmospheric pressure or a suitable under-pressure. The choice of pressure conditions may depend on the other process parameters. The formation of a uniform non-thermal electrical discharge may be facilitated by creating an under-pressure atmosphere in the chamber, preferably a moderate under-pressure of about 1-5 x 10⁴ Pa, more preferably an under-pressure of about 1.5-2.5 x 10⁴ Pa.

The gas phase may consist of any suitable gas or mixture of gases, such as air and/or a noble gas. It is preferred to include a gas in the gas phase that reacts with the plasma and the water, such as NOₓ, CO₂ or O₂.

The glow discharge plasma or the corona discharge may be an alternating current (AC) at high frequency, commonly referred to as radio frequency (RF), or direct current (DC) discharge. In a situation where the lower electrode in fact is an aqueous solution, it is preferred to use the DC mode.

In a preferred embodiment, the glow discharge plasma or the corona discharge is operated in a pulsed mode. This allows higher instantaneous powers to be applied without excessively heating the aqueous layer. In the pulsed mode, the preferred frequency is in the range of 50-5,000 Hz. Pulsed glow discharge plasma's and pulsed corona discharges are discharge types that run reliably at low and at high temperatures, in various gasses and with a wide range of compositions.

A very particular advantage of pulsed corona discharges is the fact that a highly reactive streamer discharge medium is created, while the bulk gas is at ambient temperature and pressure. Therefore, a pulsed corona promises higher efficiency than other advanced oxidation processes.

Compared with AC and radio frequency (RF) discharges, pulsed DC discharges have the advantage of causing less electromagnetic interference and providing the possibility to control the water temperature with the pulse duty cycle without changing the plasma parameters during the pulse-on times.

In another preferred embodiment, the glow discharge plasma, preferably the DC glow discharge plasma, more preferably the pulsed DC glow discharge plasma, is induced by a corona, preferably a pulsed corona. The plasma created by corona induction is a uniform and very stable, low temperature glow discharge plasma. The combination of a corona discharge and a glow discharge also provides for a highly controllable process and a highly predictable outcome of the end product quality.

Thus, it is preferred to use a pulsed DC glow discharge plasma induced by a pulsed corona, a so-called pulsed corona induced pulsed DC glow discharge plasma process. More preferably, a pulsed corona induced low frequency pulsed DC glow discharge plasma is used. Compared to pure DC or (very) high frequency DC discharges, low frequency pulsed DC discharges have the advantage of producing fewer arcs and creating a higher electron impact. This is important for producing an activated aqueous solution with a strong and long lasting biocidal activity according to this invention. A low frequency in this regard is about 50-5,000 Hz, preferably 100-1,000 Hz.

The pulsed corona induced pulsed DC glow discharge plasma process is performed at a low frequency, at a temperature below the boiling point of water and at a current, voltage and pressure sufficient to produce a stable discharge plasma, whereby the discharge plasma extends to the surface of the liquid. It is preferred to use a higher voltage than known in the art and a relatively low current, in order to avoid excessive heating of the water and to enable a more efficient formation of the biocidal solution. It shows from experience that heating of the liquid results in a destruction of the biocidally active components and actually decreases activation efficiency.

The low frequency pulsed DC glow discharge plasma is induced by a pulsed corona discharge to electrically ionize the gas above the liquid and enable the formation of ions in the liquid. The induction voltage of the corona discharge is about 5000 -20,000 V. The process starts with an initial low operating current as the non-ionized air and water form a significant dielectric barrier. Within several tens of seconds to several minutes, the current is slowly increased from about 0 mA to 100-200 mA once ionization has fully occurred, while the voltage is decreased to 1000-10,000 V, more preferably to 1000-2500 V.

The current per electrode is maintained at a rather low level in the range of 20-500 mA, preferably 100-300 mA, more preferably 100-200 mA, for a period sufficient to produce the activated water. This period typically will be dependent on parameters like the volume of the treated solution and the current density. The voltage is maintained at a level sufficient to sustain a stable discharge, at 1000 - 10, 000 V, preferably 1000-5000 V, more preferably 1000-2500 V.

The present invention shows that it is possible to produce a stable plasma by using pin shaped electrodes (as opposed to plate or annular electrodes commonly used in the art) to prevent arcing and to form a uniform and stable plasma, using a relatively low clock frequency and a corona induced plasma to electrically ionize the gas atmosphere above the water and form ions in the water, and most importantly using a relatively low current. By altering the pulse duty cycle and frequency, it was possible to control the process and heat up of the liquid and alter the properties of the treated water. The low current used and low clock frequency make the method of the invention very cost efficient and enable an economically attractive production of an activated aqueous solution.

The properties of the activated aqueous solution may be varied by altering the current, the voltage, the treatment time, the pressure and/or the electrode distance. Varying the current, electrode distance and/or pressure results in differences in current density, which is in general an important parameter for the extent of activation. Current density is a measure of the current per unit surface area of the plasma touching the liquid. The higher the current density at a constant volume, the more the liquid is activated.

In addition, the treatment time and the volume of treated water are important variables. The longer the treatment time at a constant volume or the lower the volume at a constant treatment time, the more the solution is activated.

It is further important to keep the process relatively cool, for instance to keep the temperature at a value of at the most 40 °C, preferably at 30-40°C, more preferably at 34-40°C.

In a preferred embodiment, the process of the invention is performed in a stepwise fashion, wherein the aqueous solution is subjected to at least two successive series of an activation and subsequent cooling step. Thus, the activation and cooling steps may conveniently be repeated several times, e.g. 2 to 5 times, until the desired parameters of the aqueous solution are obtained. In this way, a higher concentration of peroxides and super-oxides, a higher concentration of biocidally active ions, a higher ORP and a lower pH are obtained, resulting in a higher biocidal activity.

It was surprisingly found that the time period needed to produce a biocidal activated water was considerably reduced when a lower alcohol was added to the water.

The chemical reactivity of a glow discharge plasma or a corona discharge is based on the fact that the electric field strength at the head of the discharge streamer is extremely high. Corona and glow discharge plasmas produce hydroxyl radicals and hydrogen atoms in aqueous solutions from the dissociation and ionization of water molecules. In a humid gas phase, corona and glow discharge plasmas additionally create radicals, ions and metastables from the dissociation and ionization of the gas phase molecules or atoms. In humid air, the following main oxidizer species are produced: hydroxyl radicals, ozone, atomic oxygen, singlet oxygen and hydroperoxyl radicals. Also, small amounts of nitrogen oxides like NOₓ and N₂O are formed. By exposure of deionized water to corona or glow discharge plasmas, the electrical conductivity significantly increases. This is likely due to mainly carbonate and nitrate ions, originating from carbon(di)oxides and nitrogen(di)oxides, which are produced by the corona or the glow discharge plasma in air. Corona-induced anode metal sputtering is theoretically possible, but metals have not been identified in substantial quantities.

Through these methods, it has been determined that, during the initial stages of treatment of the aqueous solution by the electrical discharge process, a number of transformations occurs, including the formation of ions, excited water molecules and the formation of secondary electrons. These chemical reactions and actively formed radicals characterize the reactivity of the activated aqueous solution produced.

The biocidal solution produced by activation of water by the method of this invention has been shown to be unique as evidenced by its specific oxidation-reduction potential and its further parameters as described herein in the specification.

Thus, the method of the present invention provides an activated aqueous solution characterized by a peroxide content in the range of 0.01 to 0.1%, a low pH in the range of 2 to 4, an ORP in the range of 300 to 600 mV and a biocidal activity against a broad spectrum of micro-organisms. The peroxides present in the activated aqueous solution comprise hydrogen peroxide and super-peroxide compounds.

The biocidal aqueous solution preferably has an ORP of 400-600, more preferably 400-500 mV.

The pH of the biocidal solution is decreased as compared to the pH of the initial aqueous solution, due to the activation process. The acidity of the biocidal solution is not compensated, at least not fully compensated, by the presence of a "regular" anion, i.e. an anion that normally functions as a counter ion for the acid cation H⁺ or H₃O⁺, for example nitrate, sulphate, chloride, and the like. Preferably, the biocidal aqueous solution has a pH of 2.5-3.5.

The conductivity of the activated water is significantly increased as compared to the initial water. The conductivity of the activated water preferably is 300 to 600 µSiemens/cm.

Sometimes the water that is subjected to the activation process according to the invention, such as tap water, may contain chlorine-containing compounds. The pH of the obtained activated water may initially be higher than 4 and a prolonged activation may be necessary to reach a pH lower than 4.

The activated peroxide solution has substantially no available free chlorine (AFC) content. Such a solution is advantageously obtainable by the process of the invention using demineralized, distilled and/or chemically pure water, and not requiring the addition of chlorine salts to increase conductivity of the solution to be subjected to said process.

The features of the activated aqueous solution are commonly determined by standard, commercially available methods and equipment, and are preferably measured at room temperature (20 °C).

The peroxide concentration is determined by potassium permanganate titration procedure and the devices used for the measurement of pH, ORP and conductivity are a WTW 537, a Testo 230 ORP and a HANNA EC device, respectively.

Surprisingly, the activated aqueous solution has a good biocidal activity accompanied by a low cytotoxicity.

In particular, the activated aqueous solution of the second aspect is obtainable by the process of the first aspect.

Other additives may be added to the activated aqueous solution obtainable by the method of the invention in order to increase its biocidal activity or to provide the activated aqueous solution with properties suitable for its use. Examples of such additives are silver salts, e.g., silver nitrate or silver chloride, or colloidal silver; zinc salts, e.g. zinc chloride, zinc lactate, or zinc oxide; chlorhexidine; anionic, cationic, non-ionic and/or amphoteric surfactants; emulsifiers; hydrotropes; glycerol; chelating agents; alcohols; acids (organic or inorganic); bases; fragrances; coloring chemicals; or surface tension decreasing agents.

Preferably, the activated aqueous solution is stabilized with commercially available compounds known to stabilize hydrogen peroxide solutions, such as (Colloidal) Stannates, Citric Acid, Ethylenediaminetetraacetic acid (EDTA), Acetanilide, various types of Phosphonates such as the Dequest Phosphonates available from Solutia, stabilizers such as Trisodium Ethylenediamine Disuccinate available from Octel as OctaQuest E 30, or other Cation Sequestering Agents.

The present invention thus provides a composition comprising an activated aqueous solution of the invention and optional additives, as defined above.

The use of the activated aqueous solution of the invention or compositions containing said activated aqueous solution for any purpose where disinfecting and/or sanitizing and/or cleaning and/or bleaching and/or preservative activity is required, including, but not limited to, use as a bactericidal and sterilization liquid, and as cleaning, disinfection and sanitization agent.

To prepare a solution or composition for final use, it is preferred to dilute the activated aqueous solution obtainable by the process of this invention as less as possible.

In particular, the activated aqueous solution may be used for those applications where it is important to obtain disinfecting and/or sanitizing and/or cleaning and/or bleaching and/or preservative activity with the mildest agents possible, for instance domestic use, medical use, personal care, food. Also for applications where no or scarce rinsing after application is preferred, or where the solution may come into contact with food. As the activated peroxide solution in its basic form does not contain any acids or salts, no environmentally burdening residues are present. This enables the use of the solution in situations where environmentally friendly products are preferred.

Since the activated aqueous solution is non-irritating, has no odors or volatile gasses, is skin friendly, it is also optimal for situations where users do not wear any protective clothing, in cases where worker-safety has high priority or for personal application like wound disinfection or prevention of gingivitis.

Activated aqueous solution may be used in specific devices such as spray devices, spray bottles, aerosol cans, aerosol generation devices for room disinfection, and by application in the form of dipping.

### EXAMPLES

### Example 1: Solution No. I

600 milliliters of chemically pure distilled water having a pH of 6.43 and an electrical conductivity of less than 0.1 µS/cm was treated by the method of the invention. One electrode was submersed in the water and three pin shaped electrodes were placed approximately 10 mm above the water surface. Pressure was lowered and the reactor chamber cooled with cooling water. The water was then processed by pulsed corona induced low frequency pulsed DC plasma discharge under the following conditions: current increasing from 0 mA to 100 mA in 30 minutes, current was maintained at 100-120 mA for another 60 minutes of treatment, the pulsed corona induction voltage was 10 kV, the pulsed DC plasma voltage was between 1500 and 1700 Volt, pressure in the reaction chamber was 1.5x10⁴ Pa, distance between the top electrodes and water surface was 10 mm.

The resulting ORP of the solution was 420 mV, the pH of the solution was 2.97, ozone level was 0,08 mg/l and the gross concentration of hydrogen peroxide and super-peroxide compounds (measured by potassium permanganate nitration) was 215 mg/L. Electrical conductivity was 543 µSiemens/cm.

Biocidal activity: In a controlled bactericidal suspension test, a 1 ml McFarland standard 0.5 (10⁸ microorganisms per ml) was mixed 1:5 with the biocidal solution. After 20 minutes, E. coli and Bacillus cereus strains were killed 90%, while Salmonella typhimurium was killed 70%, and the yeast Rhodotorula rubra was killed 50% after 5 minutes. Thus, the solution exhibited significant biocidal activity.

For the cytotoxicity studies, the solution was tested in an ISO 10993-5, 1999: Biological Evaluation of Medical Devices - Part 5: Tests for in-vitro toxicity. The biological reactivity of a mammalian cell monolayer (L929 mouse fibroblasts) in response to the test item was determined. Positive (natural rubber extract) and negative (silicone) control (autoclaved for 30 minutes at 121°C) were used. The cultures were incubated at 37°C in a humidified atmosphere containing 5% carbon dioxide for 48 hours. The test showed that the biocidal solution exhibited only a low cytotoxicity.

### Example 2. Solution No. II

To 600 milliliters of chemically pure distilled water having a pH of 6.7 and an electrical conductivity of less than 0.1 µS/cm, 0,1% of ethanol.was added and the solution was treated by the method of the invention. One electrode was submersed in the water and three electrodes where placed approximately 10 mm above the water surface. Pressure was lowered and the reactor chamber cooled with cooling water. The water was then processed by pulsed corona induced low frequency pulsed DC plasma discharge under the following conditions: current increasing from 0 mA to 100 mA in 20 minutes, current was maintained on 100-120 mA for another 60 minutes of treatment, the pulsed corona induction voltage was 10 kV, the pulsed DC plasma voltage was between 1500 and 1600 Volt, the pressure in the reaction chamber was 1.5x10⁴ Pa, distance between the top electrodes and water surface was 10 mm.

The resulting ORP of the solution was 426 mV, the pH of the solution was 2.92, and the gross concentration of hydrogen peroxide and super-peroxide compounds was 320 mg/L. The ethanol was not detectable after treatment anymore.

The solution exhibited significant biocidal activity (measured according to Example 1). After 20 minutes, E. coli was killed 90% and Bacillus cereus strains were killed to 90%, while Salmonella typhimurium was killed 100%. The yeast Rhodotorula rubra was killed 50% after 5 minutes.

The cytotoxicity of the solution as measured according to Example 1 was low.

It can be concluded that the addition of very low levels of ethanol increases the activation efficiency significantly and produces a solution with an increased biocidal activity. The plasma process is much more stable and the plasma current increases more rapidly to its required operating values.

### Example 3. Bioburden Test

The bioburden test was applied to estimate the efficacy of the biocidal solution of the invention as sterilizing agent of soiled surgical instruments.

### (i) Production of the biocidal solution (activated water)

The water activation parameters were as follows: (1) operating voltage 1500V; (2) current per electrode 120 mA; and (3) reactor pressure 1.5 x 10⁴ Pa.

The procedure for water activation was as follows: The reactor unit is filled with approximately 500mL of demineralized water, and the distance between water surface and the electrodes was set to be approximately 10 mm. Cooling tap water was then turned on and left running for the duration of water activation and for 5 minutes thereafter.

The activated water was produced by low temperature pulsed corona induced low frequency pulsed DC plasma discharge with the following parameters: (1) 500 ml of demineralized water, conductivity ≤ 0.1 micro Siemens/cm; (2) 30 minutes at a current range from 5 to 80 mA (per electrode), electrode distance 10 mm; and (3) activation of 90 minutes in total at a current of 100-120 mA (per electrode), electrodes at 10 mm. The biocidal solution produced had a pH of 2.95, ORP was 483 mV, and hydrogen peroxide concentration was 285 mg/l.

### (ii) Bioburden Test

As a model, disposable surgical blades soiled with Bacillus subtilis were used.

The test article was a surgical blade #21 spiked with 200 CFUs of Bacillus subtilis in fetal calf serum (FCS).

The results of the treatment of the test articles with the activated water (samples C1-C3 in Table 1) in comparison with steam sterilization of the test articles (samples C4-C6 in Table 1) show that GDP-activated water and steam sterilization had the same effect on B. subtilis: both treatments have comparable sterilizing results.

**Table 1. Bioburden test on test article: Surgical blade #21 spiked with 200 CFUs of B. subtilis in FCS**

| **Test article (s)** | **CFU Score** |
|---|---|
| Blanc control | 0 |
| Positive control | >300 |
| C1: plus GDP-water | 0 |
| C2: plus GDP-water | 1 |
| C3: plus GDP-water | 0 |
| C4:sterilized | 0 |
| C5:sterilized | 0 |
| C6:sterilized | 0 |
| C7: untreated | >300 |
| C8: untreated | >300 |
| C9: untreated | >300 |

## Claims

1. A process for the preparation of a biocidal aqueous solution comprising:
(a) placing an aqueous solution in the form of a layer in a chamber suitable for a glow discharge plasma treatment,
(b) positioning an electrode in the layer of (a) and an electrode with opposite potential in the gas phase above said layer,
(c) generating a glow discharge plasma in the chamber, **characterised by**
(d) subjecting the aqueous solution to said glow discharge plasma at a voltage of 1000-10,000 V and a current per electrode of 20-500 mA, to obtain an activated aqueous solution.

2. The process according to claim 1, wherein the aqueous solution is tap, potable, demineralized, distilled and/or chemically pure water.

3. The process according to claim 1 or 2, wherein the aqueous solution contains an additive, preferably a lower alcohol.

4. The process according to any one of the claims 1-3, wherein the voltage is 1000-5000 V, preferably 1000-2500 V.

5. The process according to any one of the claims 1-4, wherein the current per electrode is 20-300 mA, preferably 100-200 mA.

6. The process according to any one of the claims 1-5, wherein an under-pressure is created in the chamber prior to generating the glow discharge plasma.

7. The process according to any one of the claims 1-6, wherein the glow discharge plasma is operated in a pulsed mode and/or is a direct current (DC) discharge plasma, preferably is a pulsed DC glow discharge plasma.

8. The process according any one of the claims 1-7, wherein the glow discharge plasma, preferably the pulsed DC glow discharge plasma, is induced by a corona, preferably a pulsed corona.

9. The process according any one of the claims 1-8, wherein the glow discharge plasma is a pulsed corona induced pulsed DC glow discharge plasma, preferably performed at a frequency in the range of 50-5,000 Hz, more preferably in the range of 100-1,000 Hz.

10. The process according to any one of the claims 1-9, wherein the layer has a depth in the range of 10 to 200 mm.

11. The process according to any one of the claims 1-10, wherein the positive electrode or anode is positioned in the gas phase above the surface of the layer and the negative electrode or cathode is positioned below the surface of the layer.

12. The process according to any one of the claims 1-11, wherein the electrodes, more preferably the anode(s), are (is) of a pin shape.

## Patentansprüche

1. Ein Verfahren zum Herstellen einer bioziden wässrigen Lösung, aufweisend:
(a) Platzieren einer wässrigen Lösung in Form einer Schicht in einer Kammer, die für eine Glimmentladungsplasmabehandlung geeignet ist,
(b) Positionieren einer Elektrode in der Schicht aus (a) und einer Elektrode mit entgegengesetztem Potential in der Gasphase über der Schicht,
(c) Erzeugen eines Glimmentladungsplasmas in der Kammer, **gekennzeichnet durch**
(d) Aussetzen der wässrigen Lösung dem Glimmentladungsplasma bei einer Spannung von 1000 V bis 10000 V und einer Stromstärke pro Elektrode von 20 mA bis 500 mA, um eine aktivierte wässrige Lösung zu erhalten.

2. Das Verfahren gemäß Anspruch 1, wobei die wässrige Lösung Leitungswasser, Trinkwasser, demineralisiertes, destilliertes und/oder chemisch reines Wasser ist.

3. Das Verfahren gemäß Anspruch 1 oder 2, wobei die wässrige Lösung einen Zusatzstoff, vorzugsweise einen niederen Alkohol enthält.

4. Das Verfahren gemäß irgendeinem der Ansprüche 1 bis 3, wobei die Spannung 1000 V bis 5000 V, vorzugsweise 1000 V bis 2500 V beträgt.

5. Das Verfahren gemäß irgendeinem der Ansprüche 1 bis 4, wobei der Strom pro Elektrode 20 mA bis 300 mA, vorzugsweise 100 mA bis 200 mA beträgt.

6. Das Verfahren gemäß irgendeinem der Ansprüche 1 bis 5, wobei vor dem Erzeugen des Glimmentladungsplasmas ein Unterdruck in der Kammer erzeugt wird.

7. Das Verfahren gemäß irgendeinem der Ansprüche 1 bis 6, wobei das Glimmentladungsplasma in einem gepulsten Modus betrieben wird und/oder ein Gleichstrom- (DC) - Entladungsplasma ist, vorzugsweise ein gepulstes Gleichstrom-Glimmentladungsplasma ist.

8. Das Verfahren gemäß irgendeinem der Ansprüche 1 bis 7, wobei das Glimmentladungsplasma, vorzugsweise das gepulste Gleichstrom-Glimmentladungsplasma durch eine Koronaentladung, vorzugsweise eine gepulste Koronaentladung induziert wird.

9. Das Verfahren gemäß irgendeinem der Ansprüche 1 bis 8, wobei das Glimmentladungsplasma ein gepulste-Koronaentladunginduziertes gepulstes Gleichstrom-Glimmentladungsplasma ist, das vorzugsweise bei einer Frequenz im Bereich von 50 Hz bis 5000 Hz, noch bevorzugter im Bereich von 100 Hz bis 1000 Hz durchgeführt wird.

10. Das Verfahren gemäß irgendeinem der Ansprüche 1 bis 9, wobei die Schicht eine Tiefe im Bereich von 10 mm bis 200 mm hat.

11. Das Verfahren gemäß irgendeinem der Ansprüche 1 bis 10, wobei die positive Elektrode oder Anode in der Gasphase über der Fläche der Schicht positioniert wird und die negative Elektrode oder Kathode unter der Fläche der Schicht positioniert wird.

12. Das Verfahren gemäß irgendeinem der Ansprüche 1 bis 11, wobei die Elektroden, noch bevorzugter die Anode(n) eine Stiftform hat (haben).

## Revendications

1. Procédé pour la préparation d'une solution aqueuse biocide comprenant le fait :
(a) de placer une solution aqueuse sous forme d'une couche dans une chambre appropriée à un traitement au plasma à décharge luminescente,
(b) de positionner une électrode dans la couche de (a) et une électrode avec un potentiel opposé dans la phase gazeuse au-dessus de ladite couche,
(c) de générer un plasma à décharge luminescente dans la chambre, **caractérisé par** le fait
(d) de soumettre la solution aqueuse audit plasma à décharge luminescente à une tension de 1000 à 10000 V et un courant par électrode de 20 à 500 mA, pour obtenir une solution aqueuse activée.

2. Procédé selon la revendication 1, dans lequel la solution aqueuse est une eau du robinet, eau potable, eau déminéralisée, eau distillée et/ou une eau chimiquement pure.

3. Procédé selon la revendication 1 ou 2, dans lequel la solution aqueuse contient un additif, de préférence un alcool inférieur.

4. Procédé selon l'une quelconque des revendications 1 à 3, dans lequel la tension est de 1000 à 5000 V, de préférence de 1000 à 2500 V.

5. Procédé selon l'une quelconque des revendications 1 à 4, dans lequel le courant par électrode est de 20 à 300 mA, de préférence de 100 à 200 mA.

6. Procédé selon l'une quelconque des revendications 1 à 5, dans lequel une sous-pression est créée dans la chambre avant de générer le plasma à décharge luminescente.

7. Procédé selon l'une quelconque des revendications 1 à 6, dans lequel le plasma à décharge luminescente est actionné dans un mode pulsé et/ou est un plasma à décharge à courant continu (CC), de préférence est un plasma à décharge luminescente à courant continu (CC) pulsé.

8. Procédé selon l'une quelconque des revendications 1 à 7, dans lequel le plasma à décharge luminescente, de préférence le plasma à décharge luminescente à courant continu (CC) pulsé, est induit par une couronne, de préférence une couronne pulsée.

9. Procédé selon l'une quelconque des revendications 1 à 8, dans lequel le plasma à décharge luminescente est un plasma à décharge luminescente à courant continu (CC) pulsé induit par une couronne pulsée, de préférence exécuté à une fréquence comprise dans la plage allant de 50 à 5000 Hz, plus préférentiellement dans la plage allant de 100 à 1000 Hz.

10. Procédé selon l'une quelconque des revendications 1 à 9, dans lequel la couche présente une profondeur comprise dans la plage allant de 10 à 200 mm.

11. Procédé selon l'une quelconque des revendications 1 à 10, dans lequel l'électrode positive ou l'anode est positionnée dans la phase gazeuse au-dessus de la surface de la couche et l'électrode négative ou la cathode est positionnée au-dessous de la surface de la couche.

12. Procédé selon l'une quelconque des revendications 1 à 11, dans lequel les électrodes, plus préférentiellement l'anode(s), sont (est) en forme de broche.
